# EUROPEAN PATENT APPLICATION

(11) **EP 4 524 162 A1**
(43) Date of publication of application: **19.03.2025**
(21) Application number: 23803829.3
(22) Date of filing: 10.05.2023
(51) Int. Cl.: C07K 16/40, A61P 35/00, A61P 19/02, A61P 25/28, G01N 33/574, G01N 33/68, A61K 39/00

(54) **ANTI-CHITINASE-3-LIKE PROTEIN 1 ANTIBODY**

(30) Priority: 10.05.2022 KR 20220057475
(71) Applicant: Osong Medical Innovation Foundation, Cheongju-si, Chungcheongbuk-do 28160 (KR); Chungbuk National University Industry-Academic Cooperation Foundation, Cheongju-si, Chungcheongbuk-do 28644 (KR); Hwatabio Co., Ltd, Daejeon 35241 (KR)
(72) Inventor: LEE, Joo Eon, Cheongju-si Chungcheongbuk-do 28160 (KR); KIM, Dae Young, Siheung-si Gyeonggi-do 14994 (KR); PARK, So Ra, Sejong 30064 (KR); HONG, Jin Tae, Cheongju-si Chungcheongbuk-do 28162 (KR)
(74) Representative: Jung, Minkyu
(86) International application number: PCT/KR2023/006327
(87) International publication number: WO 2023/219406

(57) **Abstract**

An anti-chitinase-3-like protein 1 antibody or antigen-binding fragment thereof of the present invention comprises a light chain variable region comprising CDRs of table 1 and a heavy chain variable region comprising CDRs of table 2.

## Description

### [Technical Field]

The present invention relates to an anti-chitinase-3-like protein 1 antibody. More specifically, the present invention relates to an antibody capable of specifically binding to chitinase-3-like protein 1 (CHI3L1) and thus can be used for the prevention or treatment of a CHI3L1-associated disease, or for the diagnosis of the disease.

### [Background Art]

Chitinase-3-like protein 1 (CHI3L1) is known to promote cancer metastasis, suppress immune function, and induce apoptosis inhibition, and is known to be significantly involved in the onset and progression of cancer diseases such as inflammatory responses and angiogenesis processes.

Although CHI3L1 has a low expression level under normal conditions, it has been reported that the expression level of CHI3L1 is significantly increased in the blood and tissues of patients with cancer or patients with inflammatory diseases, and thus its potential as a diagnostic marker for various types of diseases is emerging.

Accordingly, the present inventors sought to develop an antibody or antibody-related molecule that is capable of specifically binding to the above-described CHI3L1 and thus can be used for the prevention, treatment, or diagnosis of a CHI3L1-associated disease.

Related art documents include U.S. Patent No. 7,670,599.

### [Disclosure]

### [Technical Problem]

The main object of the present invention is to provide a novel antibody capable of specifically binding to chitinase-3-like protein 1 (CHI3L1).

Another object of the present invention is to provide a pharmaceutical composition for preventing or treating a CHI3L1-associated disease, a composition for diagnosing the disease, a kit for diagnosing the disease, and a method of providing information for diagnosing the disease by using the antibody.

Still another object of the present invention is to provide a polynucleotide, vector, and cell that can be used to produce the antibody.

### [Technical Solution]

According to one aspect of the present invention, the present invention provides an anti-chitinase-3-like protein 1 (CHI3L1) antibody or an antigen-binding fragment thereof, including a light chain variable region including CDR1 of SEQ ID NO: 1, CDR2 of SEQ ID NO: 2, and CDR3 of SEQ ID NO: 3, and a heavy chain variable region including CDR1 of SEQ ID NO: 31, CDR2 of SEQ ID NO: 32, and CDR3 of SEQ ID NO: 33; a light chain variable region including CDR1 of SEQ ID NO: 4, CDR2 of SEQ ID NO: 5, and CDR3 of SEQ ID NO: 6, and a heavy chain variable region including CDR1 of SEQ ID NO: 34, CDR2 of SEQ ID NO: 35, and CDR3 of SEQ ID NO: 36; a light chain variable region including CDR1 of SEQ ID NO: 7, CDR2 of SEQ ID NO: 8, and CDR3 of SEQ ID NO: 9, and a heavy chain variable region including CDR1 of SEQ ID NO: 37, CDR2 of SEQ ID NO: 38, and CDR3 of SEQ ID NO: 39; a light chain variable region including CDR1 of SEQ ID NO: 10, CDR2 of SEQ ID NO: 11, and CDR3 of SEQ ID NO: 12, and a heavy chain variable region including CDR1 of SEQ ID NO: 40, CDR2 of SEQ ID NO: 41, and CDR3 of SEQ ID NO: 42; a light chain variable region including CDR1 of SEQ ID NO: 13, CDR2 of SEQ ID NO: 14, and CDR3 of SEQ ID NO: 15, and a heavy chain variable region including CDR1 of SEQ ID NO: 43, CDR2 of SEQ ID NO: 44, and CDR3 of SEQ ID NO: 45; a light chain variable region including CDR1 of SEQ ID NO: 16, CDR2 of SEQ ID NO: 17, and CDR3 of SEQ ID NO: 18, and a heavy chain variable region including CDR1 of SEQ ID NO: 46, CDR2 of SEQ ID NO: 47, and CDR3 of SEQ ID NO: 48; a light chain variable region including CDR1 of SEQ ID NO: 19, CDR2 of SEQ ID NO: 20, and CDR3 of SEQ ID NO: 21, and a heavy chain variable region including CDR1 of SEQ ID NO: 49, CDR2 of SEQ ID NO: 50, and CDR3 of SEQ ID NO: 51; a light chain variable region including CDR1 of SEQ ID NO: 22, CDR2 of SEQ ID NO: 23, and CDR3 of SEQ ID NO: 24, and a heavy chain variable region including CDR1 of SEQ ID NO: 52, CDR2 of SEQ ID NO: 53, and CDR3 of SEQ ID NO: 54; a light chain variable region including CDR1 of SEQ ID NO: 25, CDR2 of SEQ ID NO: 26, and CDR3 of SEQ ID NO: 27, and a heavy chain variable region including CDR1 of SEQ ID NO: 55, CDR2 of SEQ ID NO: 56, and CDR3 of SEQ ID NO: 57; or a light chain variable region including CDR1 of SEQ ID NO: 28, CDR2 of SEQ ID NO: 29, and CDR3 of SEQ ID NO: 30, and a heavy chain variable region including CDR1 of SEQ ID NO: 58, CDR2 of SEQ ID NO: 59, and CDR3 of SEQ ID NO: 60.

According to another aspect of the present invention, the present invention provides a pharmaceutical composition for preventing or treating a CHI3L1-associated disease, including the antibody or antigen-binding fragment thereof of the present invention.

According to still another aspect of the present invention, the present invention provides a composition for diagnosing a CHI3L1-associated disease, including the antibody or antigen-binding fragment thereof of the present invention.

According to yet another aspect of the present invention, the present invention provides a kit for diagnosing a CHI3L1-associated disease, including the diagnostic composition of the present invention.

According to yet another aspect of the present invention, the present invention provides a method of providing information for diagnosing a CHI3L1-associated disease, including bringing the antibody or the antigen-binding fragment thereof into contact with a sample isolated from a test subject; and measuring a complex of the antibody or the antigen-binding fragment thereof and CHI3L1 formed by the contact.

According to yet another aspect of the present invention, the present invention provides a polynucleotide encoding the antibody or antigen-binding fragment thereof of the present invention.

According to yet another aspect of the present invention, the present invention provides a vector including the polynucleotide of the present invention.

According to yet another aspect of the present invention, the present invention provides a cell transformed with the vector of the present invention.

### [Advantageous Effects]

The antibody or antigen-binding fragment thereof of the present invention is capable of specifically binding to chitinase-3-like protein 1 (CHI3L1), and thus can be used for the prevention or treatment of a CHI3L1-associated disease, or for the diagnosis of the disease. Accordingly, by using the composition, kit, and information providing method of the present invention, a CHI3L1-associated disease can be effectively prevented, treated, or diagnosed. In addition, by using the polynucleotide, vector, and cell of the present invention, the antibody or antigen-binding fragment thereof of the present invention having the above-described excellent effects can be efficiently produced.

### [Description of Drawings]

FIG. 1A shows a schematic diagram illustrating a process of selecting antibodies exhibiting significant binding ability to human chitinase-3-like protein 1 (hCHI3L1) through phage display, and FIGS. 1B to 1D show the results of panning titration for Fab-I and Fab-II, respectively.
FIG. 2 shows the results of investigating the amino acid length distribution of the heavy chain CDR3 (HCDR3) of 10 antibodies against hCHI3L1 (A5, A7, D2, F2, E7, F4, H1, 1E12, C5, and H7) selected from phage display.
FIG. 3A shows the results of sodium dodecyl sulfate-polyacrylamide gel electrophoresis (SDS-PAGE) for confirming IgG production from 10 clones (A5, A7, D2, F2, E7, F4, H1, 1E12, C5, and H7) having antibodies against hCHI3L 1, and FIG. 3B shows the results of measuring the amount of IgG production.
FIGS. 4A and 4B show the results of measuring EC₅₀ values through enzyme-linked immunosorbent assay (ELISA) for 10 antibodies against hCHI3L1.
FIG. 5 shows the results of measuring the migrated cell number after treating A549 cell line with A5, A7, D2, F2, E7, F4, H1, 1E12, C5, and H7, which are antibodies against hCHI3L1, as candidate antibodies.

### [Modes of the Invention]

The antibody or antigen-binding fragment thereof of the present invention, which was discovered through phage display panning performed by using chitinase-3-like protein 1 (CHI3L1) as an antigen, is capable of specifically binding to CHI3L1.

CHI3L 1, also referred to as YKL-40, is known to be involved in various cancers, cancer metastasis, immune function, and apoptosis.

The present invention provides 10 types of antibodies or antigen-binding fragments thereof.

The 10 types are named 'A5,' 'A7,' 'D2,' 'F2,' 'E7,' 'F4,' 'H1,' '1E12,' 'C5,' and 'H7,' as shown in Tables 1 to 4, and each type of antibody or antigen-binding fragment thereof has the amino acid sequences of CDR1, CDR2, and CDR3 of the light chain variable region shown in Table 1 (expressed in Table 1 as 'CDRL1,' 'CDRL2,' and 'CDRL3,' respectively, meaning complementarity-determining regions (CDRs) of the light chain variable region) and the amino acid sequences of CDR1, CDR2, and CDR3 of the heavy chain variable region shown in Table 2 (expressed in Table 2 as 'CDRH1,' 'CDRH2,' and 'CDRH3,' respectively, meaning CDRs of the heavy chain variable region).

In one embodiment, each type of antibody or antigen-binding fragment thereof has an amino acid sequence of the light chain variable region as shown in Table 3, and an amino acid sequence of the heavy chain variable region as shown in Table 4.

The antibody or antigen-binding fragment thereof of the present invention may be in various forms satisfying the conditions described above. For example, it may be an antibody selected from the group consisting of IgG, IgA, IgM, IgE, and IgD, and may be a fragment selected from the group consisting of fragment antigen binding (Fab), Fab', F(ab')2, single-chain variable fragment (scFv), single-chain fragment variable-crystallizable fragment (scFv-Fc), a diabody, a minibody, and a triabody.

The antibody or antigen-binding fragment thereof of the present invention may be labeled for detection. At this time, labeling may be performed, for example, by direct or indirect binding of a detectable labeling substance.

It has been experimentally demonstrated that the antibody or antigen-binding fragment thereof of the present invention is capable of specifically binding to CHI3L 1 with high binding affinity. Therefore, the antibody or antigen-binding fragment thereof of the present invention can be used to prevent or treat a CHI3L1-associated disease, and can also be used to diagnose a CHI3L1-associated disease.

Therefore, the present invention provides a pharmaceutical composition for preventing or treating a CHI3L1-associated disease, including the antibody or antigen-binding fragment thereof of the present invention.

In addition, the present invention provides a composition for diagnosing a CHI3L1-associated disease, including the antibody or antigen-binding fragment thereof of the present invention.

In addition, the present invention provides a kit for diagnosing a CHI3L1-associated disease, including the antibody or antigen-binding fragment thereof of the present invention or the diagnostic composition of the present invention.

In addition, the present invention provides a method of providing information for diagnosing a CHI3L1-associated disease, including: bringing the antibody or the antigen-binding fragment thereof into contact with a sample isolated from a test subject; and measuring a complex of the antibody or the antigen-binding fragment thereof and CHI3L1 formed by the contact.

In one embodiment of the present invention, a CHI3L1-associated disease is cancer, cancer metastasis, arthritis, or dementia, but is not limited thereto. In one embodiment, a CHI3L1-associated disease is lung cancer metastasis.

A pharmaceutical composition for preventing or treating a CHI3L1-associated disease of the present invention includes the above-described antibody or antigen-binding fragment thereof of the present invention. The pharmaceutical composition of the present invention may include one or more types of the antibody or antigen-binding fragment thereof of the present invention, and may include all ten types. The pharmaceutical composition of the present invention may be the antibody or antigen-binding fragment thereof of the present invention itself, or in a form in which the antibody or antigen-binding fragment thereof is mixed with a pharmaceutically acceptable carrier. The pharmaceutical composition of the present invention may be administered orally or parenterally during clinical administration, and may be used in the form of a general pharmaceutical preparation. The pharmaceutical composition of the present invention may be used alone or in combination with methods using surgery, radiotherapy, hormone therapy, chemotherapy, and biological response modifiers. The pharmaceutical composition of the present invention may be administered at the same dosage as a general antibody preparation based on the antibody or antigen-binding fragment thereof of the present invention contained therein. For example, the pharmaceutical composition may be administered in an amount of about 1 to 10 mg per 1 kg of body weight based on the antibody or antigen-binding fragment thereof of the present invention, but the range may vary depending on the weight, age, sex, health conditions, diet, administration time, administration method, excretion rate, and disease severity of the administration subject. When clinically administered, it may be formulated into various oral or parenteral dosage forms, and at this time, diluents or excipients such as fillers, extenders, binders, wetting agents, disintegrants, and surfactants may be used.

The composition for diagnosing a CHI3L1-associated disease of the present invention includes the above-described antibody or antigen-binding fragment thereof of the present invention. The diagnostic composition of the present invention may also, like the pharmaceutical composition, include one or more types of the antibody or antigen-binding fragment thereof of the present invention, and may include all ten types. The diagnostic composition of the present invention may include an antibody or an antigen-binding fragment thereof other than the antibody or antigen-binding fragment thereof of the present invention. In addition, a substance necessary for a binding reaction between the antibody or antigen-binding fragment thereof of the present invention and an antigen, such as a reagent, may be further included. In addition, a substance necessary for detecting the binding, a substance for stabilizing the antibody or antigen-binding fragment thereof of the present invention, and the like may be further included.

The kit for diagnosing a CHI3L1-associated disease of the present invention includes the diagnostic composition of the present invention. In addition to the diagnostic composition of the present invention, the diagnostic kit of the present invention may further include reagents, devices, and the like that are necessary for diagnosing a CHI3L1-associated disease using the diagnostic composition of the present invention, such as reagents and devices necessary for detecting binding of the antibody or antigen-binding fragment thereof of the present invention to an antigen.

The method for providing information diagnosing a CHI3L1-associated disease of the present invention includes bringing the antibody or antigen-binding fragment thereof of the present invention into contact with a sample isolated from a test subject; and measuring a complex of the antibody or the antigen-binding fragment thereof and CHI3L1 formed by the contact. In the information providing method of the present invention, the test subject may be a mammal, and is preferably a human. In the information providing method of the present invention, the sample may be a biological sample, for example, a sample of tissue, a cell, a body fluid, or the like isolated from the test subject, and may be blood or serum. In the information providing method of the present invention, the measurement of the complex may be achieved using a conventional method used to measure a binding complex between an antibody or antibody-related molecule and an antigen.

The present invention also provides a polynucleotide encoding the antibody or antigen-binding fragment thereof of the present invention.

In one embodiment, the polynucleotide of the present invention has the base sequences of the encoding regions of CDR1, CDR2, and CDR3 of the light chain variable region shown in Table 5 (expressed in Table 5 as 'CDRL1,' 'CDRL2,' and 'CDRL3,' meaning CDRs of the light chain variable region), and the base sequences of the encoding regions of CDR1, CDR2, and CDR3 of the heavy chain variable region shown in Table 6 (expressed in Table 6 as 'CDRH1,' 'CDRH2,' and 'CDRH3,' meaning CDRs of the heavy chain variable region).

In one embodiment, the polynucleotide encoding each type of the antibody or antigen-binding fragment thereof of the present invention has light chain variable region encoding base sequences shown in Table 7, and heavy chain variable region encoding base sequences shown in Table 8.

The present invention also provides a vector including the polynucleotide of the present invention. In one embodiment, the vector of the present invention is configured to express the antibody or antigen-binding fragment thereof encoded by the polynucleotide in a host cell. In one embodiment, the vector of the present invention includes an origin of replication, a promoter, a selectable marker, and the like suitable for replication and expression in a host cell.

The present invention also provides a cell transformed with the vector of the present invention. In one embodiment, the cell of the present invention is a cell capable of expressing the antibody or antigen-binding fragment thereof encoded by the polynucleotide included in the vector of the present invention, and a cell in which the vector of the present invention may be replicated.

The antibody or antigen-binding fragment thereof of the present invention may be produced using the amino acid sequence and base sequence information related to the antibody or the antigen-binding fragment thereof, the polynucleotide, the vector, or the cell of the present invention.

**[Table 1]**

| ANTIBODY | CDRL1 | CDRL2 | CDRL3 |
|---|---|---|---|
| A5 | RASQSISRWLN (SEQ ID NO: 1) | ATSTLQS (SEQ ID NO: 2) | QQSYSFPWT (SEQ ID NO: 3) |
| A7 | RASQDISNYLN (SEQ ID NO: 4) | AASSLQS (SEQ ID NO: 5) | QQSYSFPYT (SEQ ID NO: 6) |
| D2 | RASQSISRYLN (SEQ ID NO: 7) | AASSLQS (SEQ ID NO: 8) | QQSSSTPWT (SEQ ID NO: 9) |
| F2 | RASQDISSWLN (SEQ ID NO: 10) | AASRLQS (SEQ ID NO: 11) | QQAYSFPWT (SEQ ID NO: 12) |
| E7 | RASQSISNWLN (SEQ ID NO: 13) | ATSRLQS (SEQ ID NO: 14) | QQSYSFPYT (SEQ ID NO: 15) |
| F4 | RASQSISRWLN (SEQ ID NO: 16) | AASRLQS (SEQ ID NO: 17) | QQSYSTPWT (SEQ ID NO: 18) |
| H1 | RASQSISNWLN (SEQ ID NO: 19) | ATSRLQS (SEQ ID NO: 20) | QQSYSFPLT (SEQ ID NO: 21) |
| 1E12 | RASQSISNYLN (SEQ ID NO: 22) | ATSTLHS (SEQ ID NO: 23) | QQSYSFPWT (SEQ ID NO: 24) |
| C5 | RASQDISRWLN (SEQ ID NO: 25) | AASTLQS (SEQ ID NO: 26) | QQSYSFPLT (SEQ ID NO: 27) |
| H7 | RASQSISSWLN (SEQ ID NO: 28) | AASRLQS (SEQ ID NO: 29) | QQTYSFPWT (SEQ ID NO: 30) |

**[Table 2]**

| ANTIBODY | CDRH1 | CDRH2 | CDRH3 |
|---|---|---|---|
| A5 | SYGMS (SEQ ID NO: 31) | AIKGSGGSTYYADSVKG (SEQ ID NO: 32) | TYGGEIDY (SEQ ID NO: 33) |
| A7 | DYAMS (SEQ ID NO: 34) | GISSSGGTIYYADSVKG (SEQ ID NO: 35) | AIGSWADI (SEQ ID NO: 36) |
| D2 | SYAMH (SEQ ID NO: 37) | AISSSSGTIYYADSVKG (SEQ ID NO: 38) | DFGAVFDY (SEQ ID NO: 39) |
| F2 | SYAMH (SEQ ID NO: 40) | SIKSSGGSKNYADSVKG (SEQ ID NO: 41) | GYGASFDF (SEQ ID NO: 42) |
| E7 | SYAMH (SEQ ID NO: 43) | AISSSGGS1YYADSVKG (SEQ ID NO: 44) | QGLSFDI (SEQ ID NO: 45) |
| F4 | SYAMS (SEQ ID NO: 46) | AISGSSGSTYYADSVKG (SEQ ID NO: 47) | AQGTSADF (SEQ ID NO: 48) |
| H1 | SYAMS (SEQ ID NO: 49) | AIRSSGGTTYYADSVKG (SEQ ID NO: 50) | SQSTVADY (SEQ ID NO: 51) |
| 1E12 | SYAMH (SEQ ID NO: 52) | AISSSGGTKYYADSVKG (SEQ ID NO: 53) | GLQGGAGAFDI (SEQ ID NO: 54) |
| C5 | SYAMS (SEQ ID NO: 55) | SISSSSGTTHYADSVKG (SEQ ID NO: 56) | GGAFDY (SEQ ID NO: 57) |
| H7 | SYGMH (SEQ ID NO: 58) | GISSSGGSKYYADSVKG (SEQ ID NO: 59) | HGHQQMDF (SEQ ID NO: 60) |

**[Table 3]**

| ANTIBODY | AMINO ACID SEQUENCE OF LIGHT CHAIN VARIABLE REGION OF ANTIBODY (CDR: UNDERLINED) |
|---|---|
| A5 | DIQMTQSPSSLSASVGDRVTITCRASQSISRWLN WYQQKPGKAPKLLIY ATSTLQS GVPSRFSGSGSGTDFTLTISSLQPEDFATYYC QQSYSFPWT FGQGTKVEIK (SEQ ID NO: 61) |
| A7 | DIQMTQSPSSLSASVGDRVTITCRASQDISNYLN WYQQKPGKAPKLLIY AASSLQS GVPSRFSGSGSGTDFTLTISSLQPEDFATYYC QQSYSFPYT FGQGTKVEIK (SEQ ID NO: 62) |
| D2 | DIQMTQSPSSLSASVGDRVTITCRASQSISRYLN WYQQKPGKAPKLLIY AASSLQS GVPSRFSGSGSGTDFTLTISSLQPEDFATYYC QQSSSTPWT FGQGTKVEIK (SEQ ID NO: 63) |
| F2 | DIQMTQSPSSLSASVGDRVTITCRASQDISSWLN WYQQKPGKAPKLLIY AASRLQS GVPSRFSGSGSGTDFTLTISSLQPEDFATYYC QQAYSFPWT FGQGTKVEIK (SEQ ID NO: 64) |
| E7 | DIQMTQSPSSLSASVGDRVTITCRASQSISNWLN WYQQKPGKAPKLLIY ATSRLQS GVPSRFSGSGSGTDFTLTISSLQPEDFATYYC QQSYSFPYT FGQGTKVEIK (SEQ ID NO: 65) |
| F4 | DIQMTQSPSSLSASVGDRVTITCRASQSISRWLN WYQQKPGKAPKLLIY AASRLQS GVPSRFSGSGSGTDFTLTISSLQPEDFATYYC QQSYSTPWT FGQGTKVEIK (SEQ ID NO: 66) |
| H1 | DIQMTQSPSSLSASVGDRVTITCRASQSISNWLN WYQQKPGKAPKLLIY ATSRLQS GVPSRFSGSGSGTDFTLTISSLQPEDFATYYC QQSYSFPLT FGQGTKVEIK (SEQ ID NO: 67) |
| 1E12 | DIQMTQSPSSLSASVGDRVTITCRASQSISNYLN WYQQKPGKAPKLLIY ATSTLHS GVPSRFSGSGSGTDFTLTISSLQPEDFATYYC QQSYSFPWT FGQGTKVEIK (SEQ ID NO: 68) |
| C5 | DIQMTQSPSSLSASVGDRVTITCRASQDISRWLN WYQQKPGKAPKLLIY AASTLQS GVPSRFSGSGSGTDFTLTISSLQPEDFATYYC QQSYSFPLT FGQGTKVEIK (SEQ ID NO: 69) |
| H7 | DIQMTQSPSSLSASVGDRVTITCRASQSISSWLN WYQQKPGKAPKLLIY AASRLQS GVPSRFSGSGSGTDFTLTISSLQPEDFATYYC QQTYSFPWT FGQGTKVEIK (SEQ ID NO: 70) |

**[Table 4]**

| ANTIBODY | AMINO ACID SEQUENCE OF HEAVY CHAIN VARIABLE REGION OF ANTIBODY (CDR: UNDERLINED) |
|---|---|
| A5 | EVQLVESGGGLVQPGGSLRLSCAASGFTFS SYGMS WVRQAPGKGLEWVS AIKGSGGSTYYADSVKG RFTISRDNSKNTLYLQMNSLRAEDTAVYYCAK TYGGEIDY WGQGTLVTVSS (SEQ ID NO: 71) |
| A7 | EVQLVESGGGLVQPGGSLRLSCAASGFTFS DYAMS WVRQAPGKGLEWVS GISSSGGTIYYADSVKG RFTISRDNSKNTLYLQMNSLRAEDTAVYYCAK AIGSWADI WGQGTLVTVSS (SEQ ID NO: 72) |
| D2 | EVQLVESGGGLVQPGGSLRLSCAASGFTFS SYAMHWVRQAPGKGLEWVS AISSSSGTIYYADSVKG RFTISRDNSKNTLYLQMNSLRAEDTAVYYCAK DFGAVFDY WGQGTLVTVSS (SEQ ID NO: 73) |
| F2 | EVQLVESGGGLAQPGGSLRLSCAASGFTFS SYAMHWVRQAPGKGLEWVS SIKSSGGSKNYADSVKG RFTISRDNSKNTLYLQMNSLRAEDTAVYYCAK GYGASFDF WGQGTLVTVSS (SEQ ID NO: 74) |
| E7 | EVQLVESGGGLVQPGGSLRLSCAASGFTFS SYAMHWVRQAPGKGLEWVS AISSSGGSIYYADSVKG RFTISRDNSKNTLYLQMNSLRAEDTAVYYCAK QGLSFDI WGQGTLVTVSS (SEQ ID NO: 75) |
| F4 | EVQLVESGGGLVQPGGSLRLSCAASGFTFS SYAMS WVRQAPGKGLEWVS AISGSSGSTYYADSVKG RFTISRDNSKNTLYLQMNSLRAEDTAVYYCAK AQGTSADF WGQGTLVTVSS (SEQ ID NO: 76) |
| H1 | EVQLVESGGGLVQPGGSLRLSCAASGFTFS SYAMS WVRQAPGKGLEWVS AIRSSGGTTYYADSVKG RFTISRDNSKNTLYLQMNSLRAEDTAVYYCAK SQSTVADY WGQGTLVTVSS (SEQ ID NO: 77) |
| 1E12 | EVQLVESGGGLVQPGGSLRLSCAASGFTFS SYAMHWVRQAPGKGLEWVS AISSSGGTKYYADSVKG RFTISRDNSKNTLYLQMNSLRAEDTAVYYCAK GLQGGAGAFDI WGQGTLVTVSS (SEQ ID NO: 78) |
| C5 | EVQLVESGGGLVQPGGSLRLSCAASGFTFS SYAMS WVRQAPGKGLEWVS SISSSSGTTHYADSVKGRFTISRDNSKNTLYLQMNSLRAEDTAVYYCAK GGAFDY WGQGTLVTVSS (SEQ ID NO: 79) |
| H7 | EVQLVESGGGLVQPGGSLRLSCAASGFTFS SYGMHWVRQAPGKGLEWVS GISSSGGSKYYADSVKGRFTISRDNSKNTLYLQMNSLRAEDTAVYYCAK HGHQQMDF WGQGTLVTVSS (SEQ ID NO: 80) |

**[Table 5]**

| ANTIBODY | CDRL1 | CDRL2 | CDRL3 |
|---|---|---|---|
| A5 | CGCGCTAGCCAGTCTATCT CTCGTTGGCTGAAC (SEQ ID NO: 81) | GCAACTTCCACTCTG CAGTCT(SEQ ID NO:82) | CAGCAATCTTACTCTTTTC CGTGGACG(SEQ ID NO: 83) |
| A7 | CGCGCTAGCCAGGATATCT CTAATTACCTGAAC (SEQ ID NO: 84) | GCAGCATCCTCTCTG CAGTCT(SEQ ID NO:85) | CAGCAATCTTACTCTTTTC CGTACACG(SEQ ID NO:86) |
| D2 | CGCGCTAGCCAGTCTATCT CTCGTTACCTGAAC (SEQ ID NO: 87) | GCAGCATCCTCTCTG CAGTCT(SEQ ID NO:88) | CAGCAATCTTCTTCTACTC CGTGGACG(SEQ ID NO:89) |
| F2 | CGCGCTAGCCAGGATATCT CTTCTTGGCTGAAC (SEQ ID NO: 90) | GCAGCATCCCGTCTG CAGTCT(SEQ ID NO:91) | CAGCAAGCATACTCTTTTC CGTGGACG(SEQ ID NO: 92) |
| E7 | CGCGCTAGCCAGTCTATCT CTAATTGGCTGAAC (SEQ ID NO: 93) | GCAACTTCCCGTCTG CAGTCT(SEQ ID NO:94) | CAGCAATCTTACTCTTTTC CGTACACG(SEQ ID NO:95) |
| F4 | CGCGCTAGCCAGTCTATCT CTCGTTGGCTGAAC (SEQ ID NO: 96) | GCAGCATCCCGTCTG CAGTCT(SEQ ID NO:97) | CAGCAATCTTACTCTACTC CGTGGACG(SEQ ID NO:98) |
| H1 | CGCGCTAGCCAGTCTATCT CTAATTGGCTGAAC (SEQ ID NO: 99) | GCAACTTCCCGTCTG CAGTCT (SEQ ID NO:100) | CAGCAATCTTACTCTTTTC CGCTGACG(SEQ ID NO: 101) |
| 1E12 | CGCGCTAGCCAGTCTATCT CTAATTACCTGAAC (SEQ ID NO: 102) | GCAACTTCCACTCTG CACTCT(SEQ ID NO:103) | CAGCAATCTTACTCTTTTC CGTGGACG(SEQ ID NO:104) |
| C5 | CGCGCTAGCCAGGATATCT CTCGTTGGCTGAAC (SEQ ID NO: 105) | GCAGCATCCACTCTG CAGTCT(SEQ ID NO:106) | CAGCAATCTTACTCTTTTC CGCTGACG (SEQ ID NO: 107) |
| H7 | CGCGCTAGCCAGTCTATCT CTTCTTGGCTGAAC (SEQ ID NO: 108) | GCAGCATCCCGTCTG CAGTCT(SEQ ID NO:109) | CAGCAAACTTACTCTTTTC CGTGGACG(SEQ ID NO: 110) |

**[Table 6]**

| ANTIBODY | CDRH1 | CDRH2 | CDRH3 |
|---|---|---|---|
| A5 | TCTTATGGTATG TCT(SEQ ID NO: 111) | GCAATCAAAGGTTCTGGTGGTTC TACTTACTATGCCGATTCAGTGA AGGGT(SEQ ID NO: 112) | ACTTACGGTGGTGAAAT CGATTAC(SEQ ID NO: 113) |
| A7 | GATTATGCAATG TCT (SEQ ID NO: 114) | GGTATCTCTTCTTCTGGTGGTACT ATCTACTATGCCGATTCAGTGAA GGGT(SEQ ID NO: 115) | GCAATCGGTTCTTGGGC AGATATC(SEQ ID NO: 116) |
| D2 | TCTTATGCAATG CAC(SEQ ID NO: 117) | GCAATCTCTTCTTCTTCTGGTACT ATCTACTATGCCGATTCAGTGAA GGGT(SEQ ID NO: 118) | GATTTCGGTGCAGTTTT CGATTAC(SEQ ID NO: 119) |
| F2 | TCTTATGCAATG CAC(SEQ ID NO: 120) | TCTATCAAATCTTCTGGTGGTTC TAAAAATTATGCCGATTCAGTGA AGGGT(SEQ ID NO: 121) | GGTTACGGTGCATCTTT CGATTTC(SEQ ID NO: 122) |
| E7 | TCTTATGCAATG CAC (SEQ ID NO: 123) | GCAATCTCTTCTTCTGGTGGTTCT ATCTACTATGCCGATTCAGTGAA GGGT(SEQ ID NO: 124) | CAGGGTCTGTCTTTCGA TATC(SEQ ID NO: 125) |
| F4 | TCTTATGCAATG TCT(SEQ ID NO: 126) | GCAATCTCTGGTTCTTCTGGTTCT ACTTACTATGCCGATTCAGTGAA GGGT(SEQ ID NO: 127) | GCACAGGGTACTTCTGC AGATTTC(SEQ ID NO: 128) |
| H1 | TCTTATGCAATG TCT(SEQ ID NO: 129) | GCAATCCGTTCTTCTGGTGGTACT ACTTACTATGCCGATTCAGTGAA GGGT(SEQ ID NO: 130) | TCTCAGTCTACTGTTGC AGATTAC(SEQ ID NO: 131) |
| 1E12 | TCTTATGCAATG CAC(SEQ ID NO: 132) | GCAATCTCTTCTTCTGGTGGTACT AAATACTATGCCGATTCAGTGAA GGGT(SEQ ID NO: 133) | GGTCTGCAGGGTGGTGC AGGTGCATTTGATATC (SEQ ID NO: 134) |
| C5 | TCTTATGCAATG TCT(SEQ ID NO: 135) | TCTATCTCTTCTTCTTCTGGTACT ACTCACTATGCCGATTCAGTGAA GGGT(SEQ ID NO: 136) | GGTGGTGCATTCGATTA C(SEQ ID NO: 137) |
| H7 | TCTTATGGTATG CAC(SEQ ID NO: 138) | GGTATCTCTTCTTCTGGTGGTTCT AAATACTATGCCGATTCAGTGAA GGGT(SEQ ID NO: 139) | CATGGTCATCAGCAGAT GGATTTC(SEQ ID NO: 140) |

**[Table 7]**

| ANTIBODY | BASE SEQUENCE OF LIGHT CHAIN VARIABLE REGION OF ANTIBODY (CDR: UNDERLINED) |
|---|---|
| A5 | GACATTCAAATGACGCAGAGTCCCTCCTCACTGAGTGCTAGCGTGGGCGATCGTGTGACAATTACTT GTCGCGCTAGCCAGTCTATCTCTCGTTGGCTGAACTGGTATCAGCAGAAACCGGGCAAGGCGCCAAA ATTGCTGATTTACGCAACTTCCACTCTGCAGTCTGGTGTACCGTCCCGTTTCTCTGGCAGCGGTTCTG GTACGGATTTTACCCTGACCATCTCAAGCCTCCAGCCTGAAGATTTTGCCACCTATTATTGTCAGCA ATCTTACTCTTTTCCGTGGACGTTCGGGCAGGGAACTAAAGTGGAAATTAAA(SEQ ID NO:141) |
| A7 | GACATTCAAATGACGCAGAGTCCCTCCTCACTGAGTGCTAGCGTGGGCGATCGTGTGACAATTACTT GTCGCGCTAGCCAGGATATCTCTAATTACCTGAACTGGTATCAGCAGAAACCGGGCAAGGCGCCAAA ATTGCTGATTTACGCAGCATCCTCTCTGCAGTCTGGTGTACCGTCCCGTTTCTCTGGCAGCGGTTCTG GTACGGATTTTACCCTGACCATCTCAAGCCTCCAGCCTGAAGATTTTGCCACCTATTATTGTCAGCA ATCTTACTCTTTTCCGTACACGTTCGGGCAGGGAACTAAAGTGGAAATTAAA(SEQ ID NO:142) |
| D2 | GACATTCAAATGACGCAGAGTCCCTCCTCACTGAGTGCTAGCGTGGGCGATCGTGTGACAATTACTT GTCGCGCTAGCCAGTCTATCTCTCGTTACCTGAACTGGTATCAGCAGAAACCGGGCAAGGCGCCAAA ATTGCTGATTTACGCAGCATCCTCTCTGCAGTCTGGTGTACCGTCCCGTTTCTCTGGCAGCGGTTCTG GTACGGATTTTACCCTGACCATCTCAAGCCTCCAGCCTGAAGATTTTGCCACCTATTATTGTCAGCA ATCTTCTTCTACTCCGTGGACGTTCGGGCAGGGAACTAAAGTGGAAATTAAA(SEQ ID NO:143) |
| F2 | GACATTCAAATGACGCAGAGTCCCTCCTCACTGAGTGCTAGCGTGGGCGATCGTGTGACAATTACTT GTCGCGCTAGCCAGGATATCTCTTCTTGGCTGAACTGGTATCAGCAGAAACCGGGCAAGGCGCCAAA ATTGCTGATTTACGCAGCATCCCGTCTGCAGTCTGGTGTACCGTCCCGTTTCTCTGGCAGCGGTTCTG GTACGGATTTTACCCTGACCATCTCAAGCCTCCAGCCTGAAGATTTTGCCACCTATTATTGTCAGCA AGCATACTCTTTTCCGTGGACGTTCGGGCAGGGAACTAAAGTGGAAATTAAA (SEQ ID NO:144) |
| E7 | GACATTCAAATGACGCAGAGTCCCTCCTCACTGAGTGCTAGCGTGGGCGATCGTGTGACAATTACTT GTCGCGCTAGCCAGTCTATCTCTAATTGGCTGAACTGGTATCAGCAGAAACCGGGCAAGGCGCCAAA ATTGCTGATTTACGCAACTTCCCGTCTGCAGTCTGGTGTACCGTCCCGTTTCTCTGGCAGCGGTTCTG GTACGGATTTTACCCTGACCATCTCAAGCCTCCAGCCTGAAGATTTTGCCACCTATTATTGTCAGCA ATCTTACTCTTTTCCGTACACGTTCGGGCAGGGAACTAAAGTGGAAATTAAA(SEQ ID NO:145) |
| F4 | GACATTCAAATGACGCAGAGTCCCTCCTCACTGAGTGCTAGCGTGGGCGATCGTGTGACAATTACTT GTCGCGCTAGCCAGTCTATCTCTCGTTGGCTGAACTGGTATCAGCAGAAACCGGGCAAGGCGCCAAA ATTGCTGATTTACGCAGCATCCCGTCTGCAGTCTGGTGTACCGTCCCGTTTCTCTGGCAGCGGTTCTG GTACGGATTTTACCCTGACCATCTCAAGCCTCCAGCCTGAAGATTTTGCCACCTATTATTGTCAGCA ATCTTACTCTACTCCGTGGACGTTCGGGCAGGGAACTAAAGTGGAAATTAAA (SEQ ID NO:146) |
| H1 | GACATTCAAATGACGCAGAGTCCCTCCTCACTGAGTGCTAGCGTGGGCGATCGTGTGACAATTACTT GTCGCGCTAGCCAGTCTATCTCTAATTGGCTGAACTGGTATCAGCAGAAACCGGGCAAGGCGCCAAA ATTGCTGATTTACGCAACTTCCCGTCTGCAGTCTGGTGTACCGTCCCGTTTCTCTGGCAGCGGTTCTG GTACGGATTTTACCCTGACCATCTCAAGCCTCCAGCCTGAAGATTTTGCCACCTATTATTGTCAGCA ATCTTACTCTTTTCCGCTGACGTTCGGGCAGGGAACTAAAGTGGAAATTAAA(SEQ ID NO:147) |
| 1E12 | GACATTCAAATGACGCAGAGTCCCTCCTCACTGAGTGCTAGCGTGGGCGATCGTGTGACAATTACTT GTCGCGCTAGCCAGTCTATCTCTAATTACCTGAACTGGTATCAGCAGAAACCGGGCAAGGCGCCAAA ATTGCTGATTTACGCAACTTCCACTCTGCACTCTGGTGTACCGTCCCGTTTCTCTGGCAGCGGTTCTG GTACGGATTTTACCCTGACCATCTCAAGCCTCCAGCCTGAAGATTTTGCCACCTATTATTGTCAGCA ATCTTACTCTTTTCCGTGGACGTTCGGGCAGGGAACTAAAGTGGAAATTAAA(SEQ ID NO:148) |
| C5 | GACATTCAAATGACGCAGAGTCCCTCCTCACTGAGTGCTAGCGTGGGCGATCGTGTGACAATTACTT GTCGCGCTAGCCAGGATATCTCTCGTTGGCTGAACTGGTATCAGCAGAAACCGGGCAAGGCGCCAAA ATTGCTGATTTACGCAGCATCCACTCTGCAGTCTGGTGTACCGTCCCGTTTCTCTGGCAGCGGTTCTG GTACGGATTTTACCCTGACCATCTCAAGCCTCCAGCCTGAAGATTTTGCCACCTATTATTGTCAGCA ATCTTACTCTTTTCCGCTGACGTTCGGGCAGGGAACTAAAGTGGAAATTAAA(SEQ ID NO:149) |
| H7 | GACATTCAAATGACGCAGAGTCCCTCCTCACTGAGTGCTAGCGTGGGCGATCGTGTGACAATTACTT GTCGCGCTAGCCAGTCTATCTCTTCTTGGCTGAACTGGTATCAGCAGAAACCGGGCAAGGCGCCAAA ATTGCTGATTTACGCAGCATCCCGTCTGCAGTCTGGTGTACCGTCCCGTTTCTCTGGCAGCGGTTCTG GTACGGATTTTACCCTGACCATCTCAAGCCTCCAGCCTGAAGATTTTGCCACCTATTATTGTCAGCA AACTTACTCTTTTCCGTGGACGTTCGGGCAGGGAACTAAAGTGGAAATTAAA(SEQ ID NO:150) |

**[Table 8]**

| ANTIBODY | BASE SEQUENCE OF HEAVY CHAIN VARIABLE REGION OF ANTIBODY (CDR: UNDERLINED) |
|---|---|
| A5 | GAAGTACAGTTGGTCGAAAGTGGCGGTGGCCTCGTGCAACCGGGTGGTTCACTGCGTCTGAGCTGCGC CGCCTCGGGTTTTACTTTCTCTTCTTATGGTATGTCTTGGGTTCGTCAGGCGCCGGGCAAGGGTCTCG AATGGGTTTCAGCAATCAAAGGTTCTGGTGGTTCTACTTACTATGCCGATTCAGTGAAGGGTCGCTT TACCATTTCCCGTGACAACTCTAAGAATACTCTGTATCTGCAGATGAACTCGCTGCGTGCCGAAGAC ACGGCCGTCTATTATTGCGCCAAAACTTACGGTGGTGAAATCGATTACTGGGGTCAGGGCACTTTAG TGACCGTCTCATCG (SEQ ID NO:151) |
| A7 | GAAGTACAGTTGGTCGAAAGTGGCGGTGGCCTCGTGCAACCGGGTGGTTCACTGCGTCTGAGCTGCGC CGCCTCGGGTTTTACTTTCTCTGATTATGCAATGTCTTGGGTTCGTCAGGCGCCGGGCAAGGGTCTCG AATGGGTTTCAGGTATCTCTTCTTCTGGTGGTACTATCTACTATGCCGATTCAGTGAAGGGTCGCTT TACCATTTCCCGTGACAACTCTAAGAATACTCTGTATCTGCAGATGAACTCGCTGCGTGCCGAAGAC ACGGCCGTCTATTATTGCGCCAAAGCAATCGGTTCTTGGGCAGATATCTGGGGTCAGGGCACTTTAG TGACCGTCTCATCG(SEQ ID NO:152) |
| D2 | GAAGTACAGTTGGTCGAAAGTGGCGGTGGCCTCGTGCAACCGGGTGGTTCACTGCGTCTGAGCTGCGC CGCCTCGGGTTTTACTTTCTCTTCTTATGCAATGCACTGGGTTCGTCAGGCGCCGGGCAAGGGTCTCG AATGGGTTTCAGCAATCTCTTCTTCTTCTGGTACTATCTACTATGCCGATTCAGTGAAGGGTCGCTT TACCATTTCCCGTGACAACTCTAAGAATACTCTGTATCTGCAGATGAACTCGCTGCGTGCCGAAGAC ACGGCCGTCTATTATTGCGCCAAAGATTTCGGTGCAGTTTTCGATTACTGGGGTCAGGGCACTTTAG TGACCGTCTCATCG (SEQ ID NO:153) |
| F2 | GAAGTACAGTTGGTCGAAAGTGGCGGTGGCCTCGCGCAACCGGGTGGTTCACTGCGTCTGAGCTGCGC CGCCTCGGGTTTTACTTTCTCTTCTTATGCAATGCACTGGGTTCGTCAGGCGCCGGGCAAGGGTCTCG AATGGGTTTCATCTATCAAATCTTCTGGTGGTTCTAAAAATTATGCCGATTCAGTGAAGGGTCGCTT TACCATTTCCCGTGACAACTCTAAGAATACTCTGTATCTGCAGATGAACTCGCTGCGTGCCGAAGAC ACGGCCGTCTATTATTGCGCCAAAGGTTACGGTGCATCTTTCGATTTCTGGGGTCAGGGCACTTTAG TGACCGTCTCATCG (SEQ ID NO:154) |
| E7 | GAAGTACAGTTGGTCGAAAGTGGCGGTGGCCTCGTGCAACCGGGTGGTTCACTGCGTCTGAGCTGCGC CGCCTCGGGTTTTACTTTCTCTTCTTATGCAATGCACTGGGTTCGTCAGGCGCCGGGCAAGGGTCTCG AATGGGTTTCAGCAATCTCTTCTTCTGGTGGTTCTATCTACTATGCCGATTCAGTGAAGGGTCGCTT TACCATTTCCCGTGACAACTCTAAGAATACTCTGTATCTGCAGATGAACTCGCTGCGTGCCGAAGAC ACGGCCGTCTATTATTGCGCCAAACAGGGTCTGTCTTTCGATATCTGGGGTCAGGGCACTTTAGTGAC CGTCTCATCG (SEQ ID NO:155) |
| F4 | GAAGTACAGTTGGTCGAAAGTGGCGGTGGCCTCGTGCAACCGGGTGGTTCACTGCGTCTGAGCTGCGC CGCCTCGGGTTTTACTTTCTCTTCTTATGCAATGTCTTGGGTTCGTCAGGCGCCGGGCAAGGGTCTCG AATGGGTTTCAGCAATCTCTGGTTCTTCTGGTTCTACTTACTATGCCGATTCAGTGAAGGGTCGCTT TACCATTTCCCGTGACAACTCTAAGAATACTCTGTATCTGCAGATGAACTCGCTGCGTGCCGAAGAC ACGGCCGTCTATTATTGCGCCAAAGCACAGGGTACTTCTGCAGATTTCTGGGGTCAGGGCACTTTAGT GACCGTCTCATCG (SEQ ID NO:156) |
| H1 | GAAGTACAGTTGGTCGAAAGTGGCGGTGGCCTCGTGCAACCGGGTGGTTCACTGCGTCTGAGCTGCGC CGCCTCGGGTTTTACTTTCTCTTCTTATGCAATGTCTTGGGTTCGTCAGGCGCCGGGCAAGGGTCTCG AATGGGTTTCAGCAATCCGTTCTTCTGGTGGTACTACTTACTATGCCGATTCAGTGAAGGGTCGCTT TACCATTTCCCGTGACAACTCTAAGAATACTCTGTATCTGCAGATGAACTCGCTGCGTGCCGAAGAC ACGGCCGTCTATTATTGCGCCAAATCTCAGTCTACTGTTGCAGATTACTGGGGTCAGGGCACTTTAG TGACCGTCTCATCG (SEQ ID NO:157) |
| 1E12 | GAAGTACAGTTGGTCGAAAGTGGCGGTGGCCTCGTGCAACCGGGTGGTTCACTGCGTCTGAGCTGCGC CGCCTCGGGTTTTACTTTCTCTTCTTATGCAATGCACTGGGTTCGTCAGGCGCCGGGCAAGGGTCTCG AATGGGTTTCAGCAATCTCTTCTTCTGGTGGTACTAAATACTATGCCGATTCAGTGAAGGGTCGCTT TACCATTTCCCGTGACAACTCTAAGAATACTCTGTATCTGCAGATGAACTCGCTGCGTGCCGAAGAC ACGGCCGTCTATTATTGCGCCAAAGGTCTGCAGGGTGGTGCAGGTGCATTTGATATCTGGGGTCAGGG TACTCTGGTTACTGTATCATCG (SEQ ID NO:158) |
| C5 | GAAGTACAGTTGGTCGAAAGTGGCGGTGGCCTCGTGCAACCGGGTGGTTCACTGCGTCTGAGCTGCGC CGCCTCGGGTTTTACTTTCTCTTCTTATGCAATGTCTTGGGTTCGTCAGGCGCCGGGCAAGGGTCTCG AATGGGTTTCATCTATCTCTTCTTCTTCTGGTACTACTCACTATGCCGATTCAGTGAAGGGTCGCTT TACCATTTCCCGTGACAACTCTAAGAATACTCTGTATCTGCAGATGAACTCGCTGCGTGCCGAAGAC ACGGCCGTCTATTATTGCGCCAAAGGTGGTGCATTCGATTACTGGGGTCAGGGCACTTTAGTGACCGT CTCATCG (SEQ ID NO:159) |
| H7 | GAAGTACAGTTGGTCGAAAGTGGCGGTGGCCTCGTGCAACCGGGTGGTTCACTGCGTCTGAGCTGCGC CGCCTCGGGTTTTACTTTCTCTTCTTATGGTATGCACTGGGTTCGTCAGGCGCCGGGCAAGGGTCTCG AATGGGTTTCAGGTATCTCTTCTTCTGGTGGTTCTAAATACTATGCCGATTCAGTGAAGGGTCGCTT TACCATTTCCCGTGACAACTCTAAGAATACTCTGTATCTGCAGATGAACTCGCTGCGTGCCGAAGAC ACGGCCGTCTATTATTGCGCCAAACATGGTCATCAGCAGATGGATTTCTGGGGTCAGGGCACTTTAG TGACCGTCTCATCG (SEQ ID NO:160) |

Hereinafter, the present invention will be described in more detail through examples. Since these examples are only intended to illustrate the present invention, the scope of the present invention is not to be construed as being limited by these examples.

### [Examples]

### Example 1. Screening of Fab antibody clones against CHI3L1

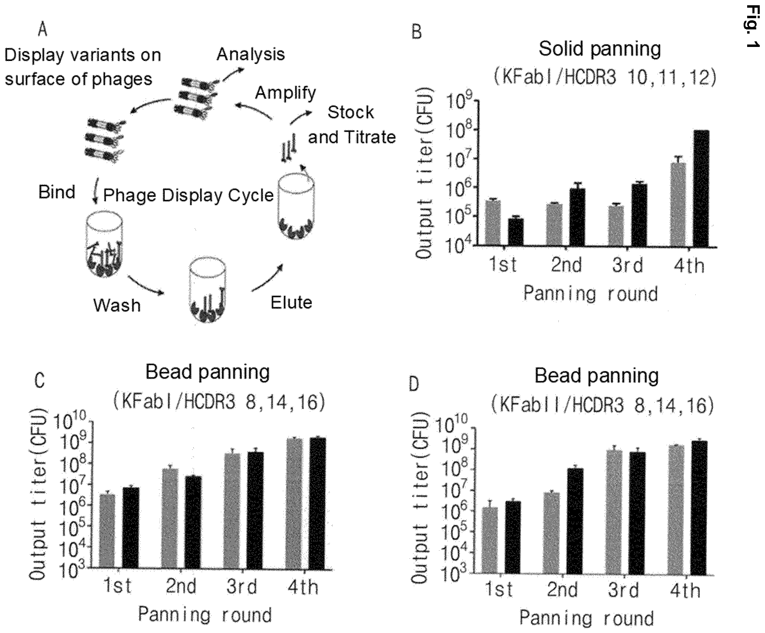

To discover antibodies that specifically bind to CHI3L1, immunotube and magnetic bead-based phage display panning was performed using CHI3L1 as an antigen, and monoclonal phage ELISA was performed to select clones that specifically bind to CHI3L1 (FIG. 1).

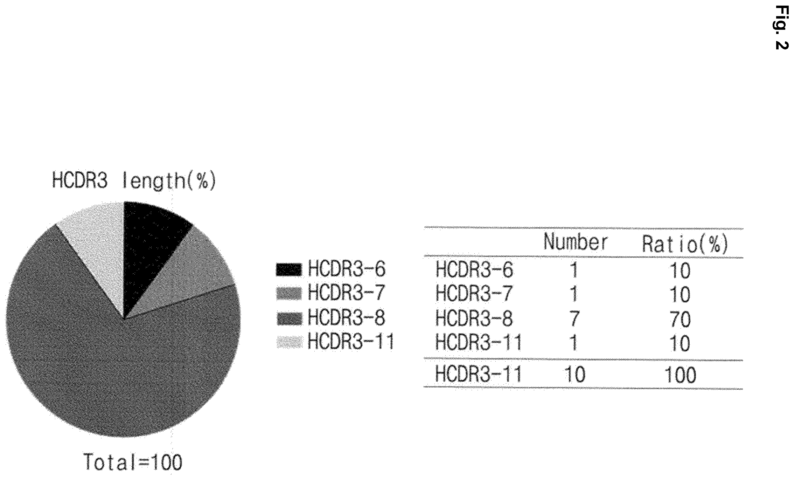

A total of 10 Fab clones were selected through base sequence analysis and amino acid sequence analysis of the clones that were found to be positive in the monoclonal phage ELISA. As a result of investigating the length distribution of the heavy chain CDR3 (HCDR3) of the selected Fab clones, it was confirmed that 70% of the clones had eight residues, and 10% of the clones respectively had 6, 7, and 11 residues (FIG. 2).

### Example 2. Expression and purification of antibodies in the form of IgG

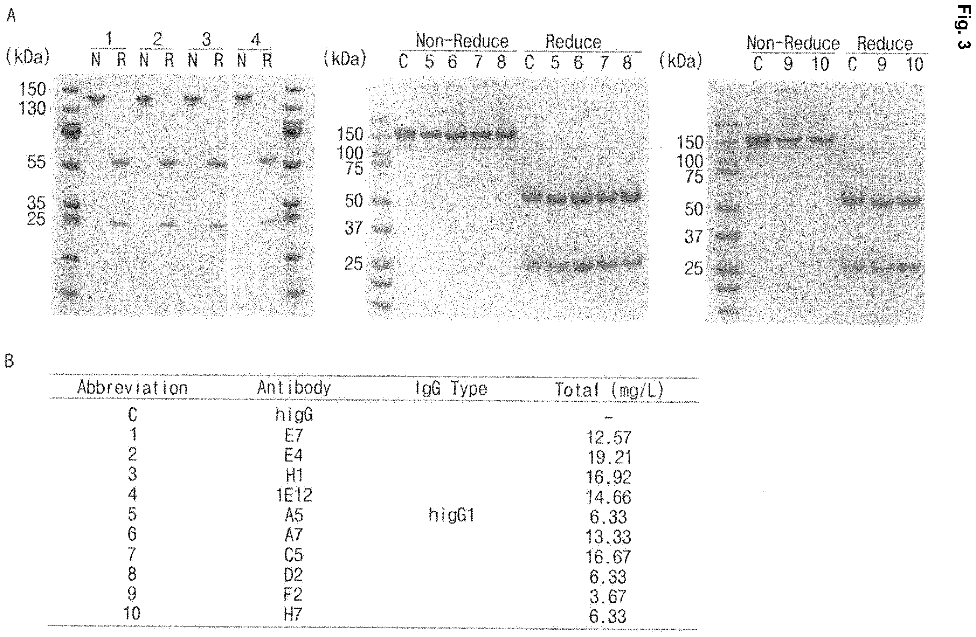

The clones selected in Example 1 were converted into the IgG form of human antibodies, and an experiment was performed to verify the results. Ten types of anti-CHI3L1 antibodies were co-transfected into ExpiCHO-S cells and cultured, and IgG was purified using a Protein A resin column. The molecular weight and purity of the purified IgG antibodies were confirmed through SDS-PAGE (FIG. 3).

### Example 3. ELISA analysis of antibodies

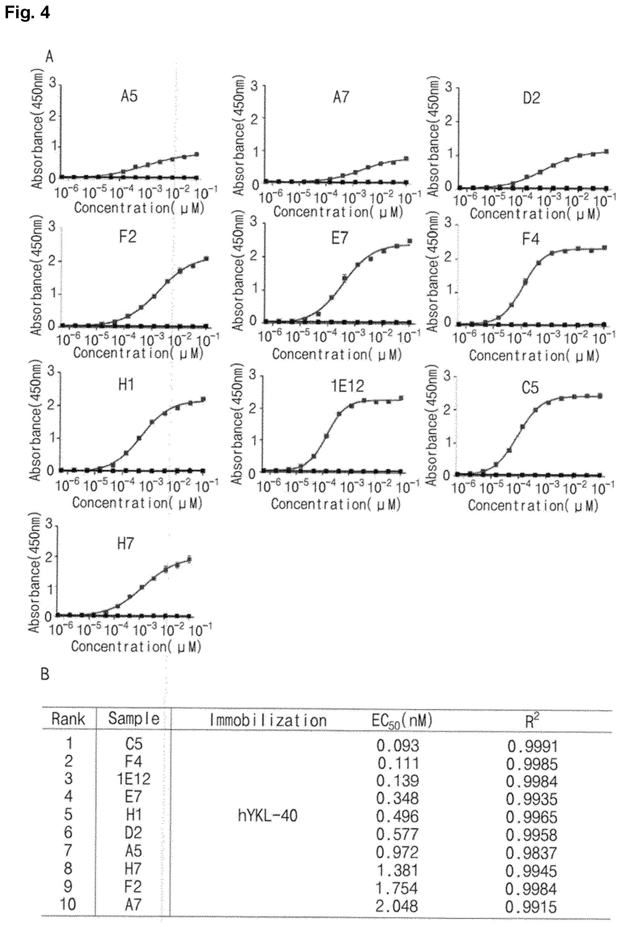

An experiment was performed to measure antigen affinity by investigating the EC₅₀ value for each antibody through ELISA. As a result, it was confirmed that all 10 antibodies bound to the CHI3L1 antigen protein. In terms of the EC₅₀ value, C5, F4, and 1E12 antibodies exhibited particularly excellent binding ability (FIG. 4).

### Example 4. Analysis of anticancer efficacy of antibodies

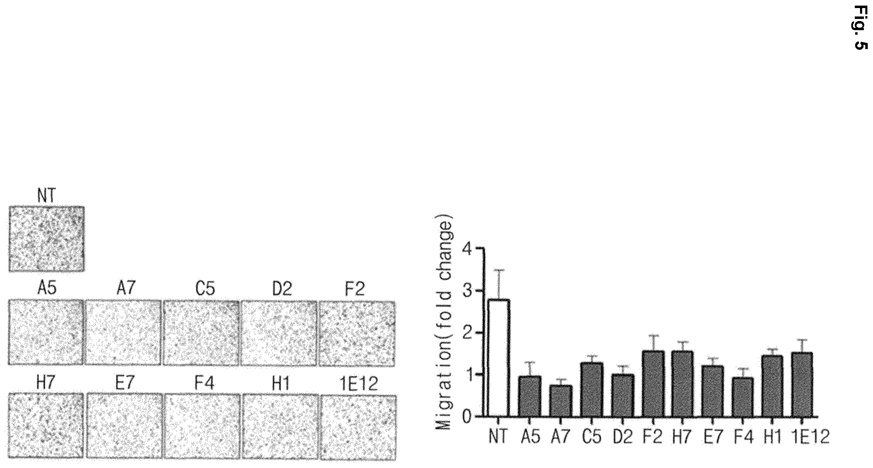

An experiment was performed to determine whether antibodies against CHI3L1 had an effect of inhibiting cancer metastasis when lung cancer cell lines were treated. The migration of the human lung cancer cell line, A549 cell line, was quantitatively investigated in a permeable insert. As a result, it was confirmed that when the 10 antibodies against human CHI3L1 (hCHI3L1) were treated, the number of migrated cells in the A549 cell line decreased compared to the control group (FIG. 5).

## Claims

1. An anti-chitinase-3-like protein 1 antibody or an antigen-binding fragment thereof, comprising:
a light chain variable region including CDR1 of SEQ ID NO: 1, CDR2 of SEQ ID NO: 2, and CDR3 of SEQ ID NO: 3, and a heavy chain variable region including CDR1 of SEQ ID NO: 31, CDR2 of SEQ ID NO: 32, and CDR3 of SEQ ID NO: 33;
a light chain variable region including CDR1 of SEQ ID NO: 4, CDR2 of SEQ ID NO: 5, and CDR3 of SEQ ID NO: 6, and a heavy chain variable region including CDR1 of SEQ ID NO: 34, CDR2 of SEQ ID NO: 35, and CDR3 of SEQ ID NO: 36;
a light chain variable region including CDR1 of SEQ ID NO: 7, CDR2 of SEQ ID NO: 8, and CDR3 of SEQ ID NO: 9, and a heavy chain variable region including CDR1 of SEQ ID NO: 37, CDR2 of SEQ ID NO: 38, and CDR3 of SEQ ID NO: 39;
a light chain variable region including CDR1 of SEQ ID NO: 10, CDR2 of SEQ ID NO: 11, and CDR3 of SEQ ID NO: 12, and a heavy chain variable region including CDR1 of SEQ ID NO: 40, CDR2 of SEQ ID NO: 41, and CDR3 of SEQ ID NO: 42;
a light chain variable region including CDR1 of SEQ ID NO: 13, CDR2 of SEQ ID NO: 14, and CDR3 of SEQ ID NO: 15, and a heavy chain variable region including CDR1 of SEQ ID NO: 43, CDR2 of SEQ ID NO: 44, and CDR3 of SEQ ID NO: 45;
a light chain variable region including CDR1 of SEQ ID NO: 16, CDR2 of SEQ ID NO: 17, and CDR3 of SEQ ID NO: 18, and a heavy chain variable region including CDR1 of SEQ ID NO: 46, CDR2 of SEQ ID NO: 47, and CDR3 of SEQ ID NO: 48;
a light chain variable region including CDR1 of SEQ ID NO: 19, CDR2 of SEQ ID NO: 20, and CDR3 of SEQ ID NO: 21, and a heavy chain variable region including CDR1 of SEQ ID NO: 49, CDR2 of SEQ ID NO: 50, and CDR3 of SEQ ID NO: 51;
a light chain variable region including CDR1 of SEQ ID NO: 22, CDR2 of SEQ ID NO: 23, and CDR3 of SEQ ID NO: 24, and a heavy chain variable region including CDR1 of SEQ ID NO: 52, CDR2 of SEQ ID NO: 53, and CDR3 of SEQ ID NO: 54;
a light chain variable region including CDR1 of SEQ ID NO: 25, CDR2 of SEQ ID NO: 26, and CDR3 of SEQ ID NO: 27, and a heavy chain variable region including CDR1 of SEQ ID NO: 55, CDR2 of SEQ ID NO: 56, and CDR3 of SEQ ID NO: 57; or
a light chain variable region including CDR1 of SEQ ID NO: 28, CDR2 of SEQ ID NO: 29, and CDR3 of SEQ ID NO: 30, and a heavy chain variable region including CDR1 of SEQ ID NO: 58, CDR2 of SEQ ID NO: 59, and CDR3 of SEQ ID NO: 60.

2. The antibody or the antigen-binding fragment thereof of claim 1, comprising:
a light chain variable region of SEQ ID NO: 61 and a heavy chain variable region of SEQ ID NO: 71;
a light chain variable region of SEQ ID NO: 62 and a heavy chain variable region of SEQ ID NO: 72;
a light chain variable region of SEQ ID NO: 63 and a heavy chain variable region of SEQ ID NO: 73;
a light chain variable region of SEQ ID NO: 64 and a heavy chain variable region of SEQ ID NO: 74;
a light chain variable region of SEQ ID NO: 65 and a heavy chain variable region of SEQ ID NO: 75;
a light chain variable region of SEQ ID NO: 66 and a heavy chain variable region of SEQ ID NO: 76;
a light chain variable region of SEQ ID NO: 67 and a heavy chain variable region of SEQ ID NO: 77;
a light chain variable region of SEQ ID NO: 68 and a heavy chain variable region of SEQ ID NO: 78;
a light chain variable region of SEQ ID NO: 69 and a heavy chain variable region of SEQ ID NO: 79; or
a light chain variable region of SEQ ID NO: 70 and a heavy chain variable region of SEQ ID NO: 80.

3. A pharmaceutical composition for preventing or treating a chitinase-3-like protein 1-associated disease, comprising the antibody or the antigen-binding fragment thereof of claim 1 or claim 2.

4. The pharmaceutical composition of claim 3, wherein the chitinase-3-like protein 1-associated disease is cancer, cancer metastasis, arthritis, or dementia.

5. The pharmaceutical composition of claim 4, wherein the chitinase-3-like protein 1-associated disease is lung cancer metastasis.

6. A composition for diagnosing a chitinase-3-like protein 1-associated disease, comprising the antibody or the antigen-binding fragment thereof of claim 1 or claim 2.

7. The composition of claim 6, wherein the chitinase-3-like protein 1-associated disease is cancer, cancer metastasis, arthritis, or dementia.

8. A kit for diagnosing a chitinase-3-like protein 1-associated disease, comprising the composition of claim 6.

9. The kit of claim 8, wherein the chitinase-3-like protein 1-associated disease is cancer, cancer metastasis, arthritis, or dementia.

10. A method of providing information for diagnosing a chitinase-3-like protein 1-associated disease, comprising:
bringing the antibody or the antigen-binding fragment thereof of claim 1 or claim 2 into contact with a sample isolated from a test subject; and
measuring a complex of the antibody or the antigen-binding fragment thereof and chitinase-3-like protein 1 formed by the contact.

11. The method of claim 10, wherein the chitinase-3-like protein 1-associated disease is cancer, cancer metastasis, arthritis, or dementia.

12. A polynucleotide encoding the antibody or the antigen-binding fragment thereof of claim 1 or claim 2.

13. The polynucleotide of claim 12, comprising:
a light chain variable region encoding sequence including a CDR1 encoding sequence of SEQ ID NO: 81, a CDR2 encoding sequence of SEQ ID NO: 82, and a CDR3 encoding sequence of SEQ ID NO: 83, and a heavy chain variable region encoding sequence including a CDR1 encoding sequence of SEQ ID NO: 111, a CDR2 encoding sequence of SEQ ID NO: 112, and a CDR3 encoding sequence of SEQ ID NO: 113;
a light chain variable region encoding sequence including a CDR1 encoding sequence of SEQ ID NO: 84, a CDR2 encoding sequence of SEQ ID NO: 85, and a CDR3 encoding sequence of SEQ ID NO: 86, and a heavy chain variable region encoding sequence including a CDR1 encoding sequence of SEQ ID NO: 114, a CDR2 encoding sequence of SEQ ID NO: 115, and a CDR3 encoding sequence of SEQ ID NO: 116;
a light chain variable region encoding sequence including a CDR1 encoding sequence of SEQ ID NO: 87, a CDR2 encoding sequence of SEQ ID NO: 88, and a CDR3 encoding sequence of SEQ ID NO: 89, and a heavy chain variable region encoding sequence including a CDR1 encoding sequence of SEQ ID NO: 117, a CDR2 encoding sequence of SEQ ID NO: 118, and a CDR3 encoding sequence of SEQ ID NO: 119;
a light chain variable region encoding sequence including a CDR1 encoding sequence of SEQ ID NO: 90, a CDR2 encoding sequence of SEQ ID NO: 91, and a CDR3 encoding sequence of SEQ ID NO: 92, and a heavy chain variable region encoding sequence including a CDR1 encoding sequence of SEQ ID NO: 120, a CDR2 encoding sequence of SEQ ID NO: 121, and a CDR3 encoding sequence of SEQ ID NO: 122;
a light chain variable region encoding sequence including a CDR1 encoding sequence of SEQ ID NO: 93, a CDR2 encoding sequence of SEQ ID NO: 94, and a CDR3 encoding sequence of SEQ ID NO: 95, and a heavy chain variable region encoding sequence including a CDR1 encoding sequence of SEQ ID NO: 123, a CDR2 encoding sequence of SEQ ID NO: 124, and a CDR3 encoding sequence of SEQ ID NO: 125;
a light chain variable region encoding sequence including a CDR1 encoding sequence of SEQ ID NO: 96, a CDR2 encoding sequence of SEQ ID NO: 97, and a CDR3 encoding sequence of SEQ ID NO: 98, and a heavy chain variable region encoding sequence including a CDR1 encoding sequence of SEQ ID NO: 126, a CDR2 encoding sequence of SEQ ID NO: 127, and a CDR3 encoding sequence of SEQ ID NO: 128;
a light chain variable region encoding sequence including a CDR1 encoding sequence of SEQ ID NO: 99, a CDR2 encoding sequence of SEQ ID NO: 100, and a CDR3 encoding sequence of SEQ ID NO: 101, and a heavy chain variable region encoding sequence including a CDR1 encoding sequence of SEQ ID NO: 129, a CDR2 encoding sequence of SEQ ID NO: 130, and a CDR3 encoding sequence of SEQ ID NO: 131;
a light chain variable region encoding sequence including a CDR1 encoding sequence of SEQ ID NO: 102, a CDR2 encoding sequence of SEQ ID NO: 103, and a CDR3 encoding sequence of SEQ ID NO: 104, and a heavy chain variable region encoding sequence including a CDR1 encoding sequence of SEQ ID NO: 132, a CDR2 encoding sequence of SEQ ID NO: 133, and a CDR3 encoding sequence of SEQ ID NO: 134;
a light chain variable region encoding sequence including a CDR1 encoding sequence of SEQ ID NO: 105, a CDR2 encoding sequence of SEQ ID NO: 106, and a CDR3 encoding sequence of SEQ ID NO: 107, and a heavy chain variable region encoding sequence including a CDR1 encoding sequence of SEQ ID NO: 135, a CDR2 encoding sequence of SEQ ID NO: 136, and a CDR3 encoding sequence of SEQ ID NO: 137; or
a light chain variable region encoding sequence including a CDR1 encoding sequence of SEQ ID NO: 108, a CDR2 encoding sequence of SEQ ID NO: 109, and a CDR3 encoding sequence of SEQ ID NO: 110, and a heavy chain variable region encoding sequence including a CDR1 encoding sequence of SEQ ID NO: 138, a CDR2 encoding sequence of SEQ ID NO: 139, and a CDR3 encoding sequence of SEQ ID NO: 140.

14. The polynucleotide of claim 13, comprising:
a light chain variable region encoding sequence of SEQ ID NO: 141 and a heavy chain variable region encoding sequence of SEQ ID NO: 151;
a light chain variable region encoding sequence of SEQ ID NO: 142 and a heavy chain variable region encoding sequence of SEQ ID NO: 152;
a light chain variable region encoding sequence of SEQ ID NO: 143 and a heavy chain variable region encoding sequence of SEQ ID NO: 153;
a light chain variable region encoding sequence of SEQ ID NO: 144 and a heavy chain variable region encoding sequence of SEQ ID NO: 154;
a light chain variable region encoding sequence of SEQ ID NO: 145 and a heavy chain variable region encoding sequence of SEQ ID NO: 155;
a light chain variable region encoding sequence of SEQ ID NO: 146 and a heavy chain variable region encoding sequence of SEQ ID NO: 156;
a light chain variable region encoding sequence of SEQ ID NO: 147 and a heavy chain variable region encoding sequence of SEQ ID NO: 157;
a light chain variable region encoding sequence of SEQ ID NO: 148 and a heavy chain variable region encoding sequence of SEQ ID NO: 158;
a light chain variable region encoding sequence of SEQ ID NO: 149 and a heavy chain variable region encoding sequence of SEQ ID NO: 159; or
a light chain variable region encoding sequence of SEQ ID NO: 150 and a heavy chain variable region encoding sequence of SEQ ID NO: 160.

15. A vector comprising the polynucleotide of claim 12.

16. A cell transformed with the vector of claim 15.
